(19) Europäisches Patentamt
European Patent Office
Office européen des brevets

(11) Veröffentlichungsnummer: **0 003 359**
**B1**

(12) # EUROPÄISCHE PATENTSCHRIFT

(21) Anmeldenummer: **79100225.6**

(22) Anmeldetag: **26.01.79**

(51) Int. Cl.³: **C 07 D 319/20, A 61 K 31/335**

(54) **Spezifische optische Isomeren von 2-Aminoäthyl-1,4-benzodioxanen, Verfahren zu ihrer Herstellung, sowie pharmazeutische Präparate enthaltend diese Verbindungen.**

(30) Priorität: **30.01.78 US 873457**

(43) Veröffentlichungstag der Anmeldung: **08.08.79 Patentblatt 79/16**

(45) Bekanntmachung des Hinweises auf die Patenterteilung: **11.02.81 Patentblatt 81/6**

(84) Benannte Vertragsstaaten: **BE CH DE FR GB IT LU NL SE**

(56) Entgegenhaltungen: **US - A - 3 312 592**

(73) Patentinhaber: **CIBA-GEIGY AG
Patentabteilung Postfach
CH-4002 Basel (CH)**

(72) Erfinder: **Gschwend, Heinz Werner, Dr.
112 Sherwood Drive
New Providence New Jersey (US)**

(72) Erfinder: **Huebner, Charles Ferdinand, Dr.
40 Edgewood Road
Chatham New Jersey (US)**

(74) Vertreter: **Zumstein sen., Fritz, Dr. et al.
Bräuhausstrasse 4
D-8000 München 2 (DE)**

EP 0 003 359 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Spezifische optische Isomeren von 2-Aminoäthyl-1,4-benzodioxanen, Verfahren zu ihrer Herstellung, sowie pharmazeutische Präparate enthaltend diese Verbindungen

Komplexe Isomerengemische von 2 - (2 - Aralkylamino - 1 - hydroxyäthyl) - 1,4 - benzodioxanen sind im US-Patent 3,312,592, in J. Med. Chem. *13*, 169(1970) oder in Chem. Abstr. *79* 126411j(1973) als $\beta$-adrenergisch blockierende, sympatholytische und adrenolytische Wirkstoffe für die Behandlung oder Vorbeugung von Krankheiten der Koronararterien offenbart. Diese Gemische von mindestens acht erythro- und threodiastereomeren Formen können nicht leicht aufgetrennt werden. Anhand von unserem neuen Verfahren zur Herstellung von geeignet substituierten erythro-Formen von 2 - Oxiranyl 1,4 - benzodioxan Zwischenprodukten ist es möglich, einfache Gemische von genannten Isomeren zu erhalten, welche viel leichter aufgetrennt werden können.

Ueberraschend hatte man gefunden, dass lediglich die R,S,R-Diastereomere der allgemeinen Formel I die genannten wertvollen pharmakologischen Eigenschaften aufweisen, während die S,R,S-S,R,R- und R,S,S-Analoga eher zu ihren Nebenwirkungen, z.B., Toxizität beitragen.

Die Erfindung betrifft spezifische Diastereomere von erythro - 2 - ( - Aralkylamino - 1 - hydroxyäthyl) - 1,4 - benzodioxanen der allgemeinen Formel I

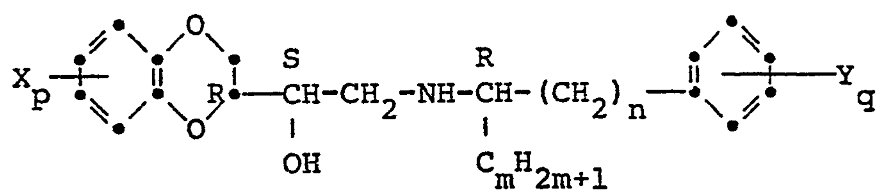 (I),

worin jedes der Symbole X und Y Wasserstoff, Niederalkyl, Niederalkoxy, Halogen oder Trifluormethyl bedeutet, jedes der Symbole p und q für die ganze Zahl 1 oder 2 steht, und jedes der Symbole m und n die ganze Zahl 1 bis 4 bedeutet, und ihre Salze, insbesondere ihre pharmazeutisch verwendbahren Salze.

Der Ausdruck "nieder" definiert in den oben oder nachfolgend genannten organischen Resten oder Verbindungen solche mit höchstens 7, vorzugsweise bis 4 Kohlenstoffatomen.

Dementsprechend ist eine Niederalkylgruppe X und/oder Y vorzugsweise Methyl, aber auch z.B. Aethyl, n- oder iso-propyl oder -Butyl. Niederalkoxy bedeutet insbesondere Methoxy, aber auch Aethoxy, n- oder iso-Propoxy oder -Butoxy, und Halogen insbesondere Fluor, Chlor oder Brom.

Die Symbole m, p and q bedeuten vorzugsweise 1 und n steht insbesondere für 1 oder 2.

Säureadditionssalze sind vorzugsweise pharmazeutisch verwendbare Säureadditionssalze von Basen der allgemeinen Formel I, welche vorzugsweise solche von starken anorganischen oder organischen Säuren sind, z.B. Säureadditionssalze mit den weiter unten aufgezählten Säuren.

Die Verbindungen der Erfindung zeigen wertvolle pharmakologische Eigenschaften, in erster Linie hypotensive, antihypertensive und bradykardische Wirkungen, welche unter anderem durch ihre $\alpha$- und $\beta$-adrenergisch blockierenden, vasodilatorischen und ihre hemmenden Effekte auf das zentrale sympatische Nervensystem zustandekommen. Diese Wirkungen können in Tierversuchen, vorzugsweise an Säugetieren, wie Ratten, Katzen oder Hunden, als Testobjekte nachgewiesen werden. Die Tiere können normotensiv oder hypertensiv, z.B. genetisch hypertensiv Ratten sein. Die genannten Verbindungen können ihnen enteral oder parenteral, vorzugsweise oral oder intravenös, z.B. durch Gelatine-Kapseln oder in Form von Stärke enthaltenden Suspensionen bzw. wässerigen Lösungen, verabreicht werden. Die verwendete Dosis kann in einem Bereich von ungefähr zwischen 0,01 und 50 mg/kg/Tag, vorzugsweise ungefähr 0,1 und 10 mg/kg/Tag, insbesondere ungefähr 1 und 5 mg/kg/Tag liegen. Die blutdrucksenkende Wirkung wird entweder direkt mit einem Katheter, der z.B. in die femurale Arterie des Hundes eingeführt ist, oder indirekt durch Sphygmomanometrie am Rattenschwanz, und einem Uebertragungsinstrument, welches den Blutdruck vor und nach der Verabreichung des Wirkstoffes in mm Hg angibt, registriert. So ist z.B. das l-erythro-2R-[2-(4-Phenyl-2R-butylamino)-1S-hydroxyäthyl]-1,4-benzodioxan, ein typischer Vertreter von Verbindungen der vorliegenden Erfindung, vorzugsweise in Form seines Hydrochlorids, sehr wirksam in genannten hypertensiven Ratten, bei peroralen Dosen von 5 mg/kg/Tag und darunter. Die bradykardischen Wirkungen dieser Verbindung können nach einer niedrigeren intravenösen Dosis von 0,03 mg/kg registreirt werden, wobei man anesthätisierte, bilateral vagotomisierte Bastardhunde, deren Herzfrequenz durch elektrische Reizung erhöht wird, verwendet. Die Verbindungen der vorliegenden Erfindung können dementsprechend als Antihypertensiva und bradykardische Mittel, z.B. in der Behandlung oder Handhabung der primären oder sekundären Hypertension, Angina pectoris und der chronischen Hypertension, eingesetzt werden. Sie können auch als Zwischenprodukte zur Herstellung von anderen wertvollen Verbindungen, insbesondere von pharmakologisch wirksamen Präparaten verwendet werden.

Bevorzugte Verbindungen sind diejenigen der Formel I, worin jedes der Symbole X und Y Wasserstoff, Fluor, Chlor, Trifluormethyl, oder Alkyl oder Alkoxy, jedes mit höchstens 4 Kohlenstoffatomen, bedeutet, p für die Zahl eins steht, q 1 oder 2 bedeutet und jedes der Symbole m und n für die Zahl 1 oder 2 steht, und ihre pharmazeutisch verwendbaren Säureadditionssalze.

Besonders hervorzuheben sind die Verbindungen der allgemeinen Formel II

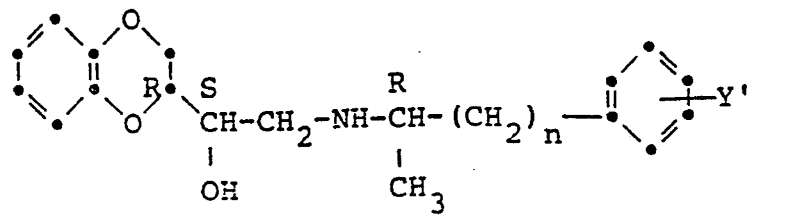

(II),

worin Y' Wasserstoff, Alkyl oder Alkoxy, jedes mit höchstens 4 Kohlenstoffatomen, bedeutet, und n für die Zahl 1 oder 2 steht, und ihre pharmazeutisch verwendbaren Säureadditionssalze.

Die Verbindungen der Erfindung können nach an sich bekannten Methoden, vorzugsweise dadurch hergestellt werden, dass man

1) Verbindungen der allgemeinen Formeln III und IV

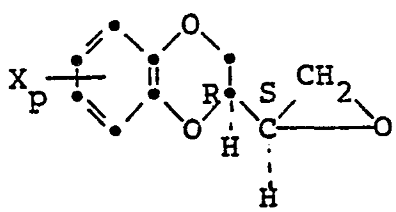

+

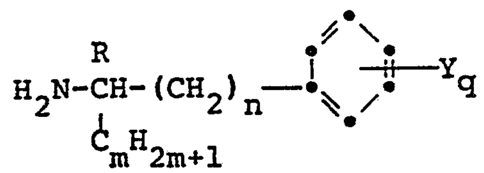

(III) (IV),

in welchen alle Symbole die oben angegebenen Bedeutungen haben, kondensiert.

Die Kondesation mit Epoxiden der allgemeinen Formel III braucht keine Kondensationsmittel, sondern eher erhöhte Temperaturen, z.B. bis 100 — 150°.

Der Ausgangstoff der Formel III wird vorzugsweise gemäss unserem neuen Verfahren, welches die folgenden Schritte umfasst, hergestellt:

a)

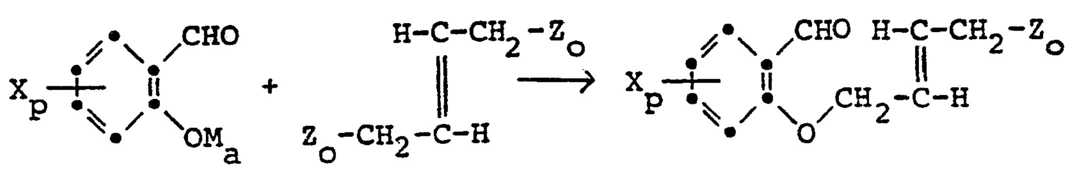

(V),

worin $M_a$ ein Alkalimetallatom bedeutet, und $Z_o$ für ein Halogenatom steht;

b)

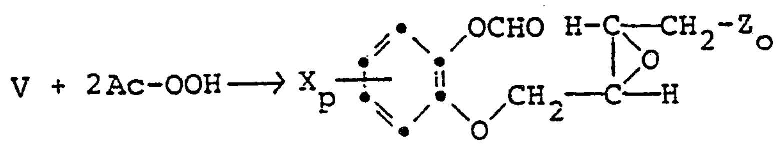

(VI),

worin der Rest Ac Niederalkanoyl, Halogen-niederalkanoyl, unsubstituiertes oder substituiertes Benzoyl oder Phthaloyl bedeutet, wobei letztere einen oder zwei Substituenten X enthalten, und

c) Ringschluss der genannten Epoxide der Formel VI in Gegenwart einer starken Base, wobei man die Epoxide der Formel III erhält.

Die starke Base wird durch die Formel $M^{\oplus}OH^{\ominus}$ charakterisiert, worin $M^{\oplus}$ das vom M abgeleitete Kation ist. Das Symbol M ist insbesondere der Rest einer quaternären organischen Stickstoff enthaltenden Verbindung oder ein Alkalimetallatom, wie Lithium, Natrium oder vorzugsweise Kalium. Der Rest einer quaternären organischen Stickstoff enthaltenden Verbindung ist z.B. ein Tetra-niederalkylammonium-, wie Tetraäthylammonium-, Tetrabutylammonium-, Trimethyl - butyl - ammonium-, aber auch ein Benzyl - triniederalkylammonium-, z.B. Benzyl-trimethylammoniumrest, oder eine analoge gestättigte heterocyclische Gruppe, z.N. ein N,N - Dimethyl - piperidinium oder N,N -

Dimethyl - pyrrolidinium- oder ein Aryl - triniederalkylammonium, z.B. ein Phenyl-trimethylammoniumrest.

In den obigen Reaktionen bedeutet $M_a$ Lithium oder Kalium, vorzugsweise Natrium, und die starke Base ist in erster Linie ein solches Metallhydroxyd, z.B. Kaliumhydroxyd oder eine starke quaternäre organische Stickstoff enthaltende Base, z.B. ein Triniederalkyl - benzyl - ammoniumhydroxyd. Das Symbol $Z_0$ bedeutet vor allem Chlor, aber auch Brom oder Jod. Die Percarbonsäure AcOOH ist z.B. die Peressig-, Trifluorperessig-, Perbenzoe-, m - Chlor - perbenzoe- oder die Monoperphthalsäure. Die Peroxydation soll in niedrigsiedenden Halogen-alkanen, z.b. Methylenchlorid für die genannten aromatischen Persäuren oder in Niederalkyl-alkanoaten für die aliphatischen Persäuren, durchgeführt werden.

Die genannten Ausgangsstoffe der allgemeinen Formell III können auch in situ, gemäss unserem neuen Verfahren hergestellt werden. Dieses besteht darin, dass man in Gegenwart einer starken Base, Verbindungen der nachfolgend angegebenen Formeln, kondensiert:

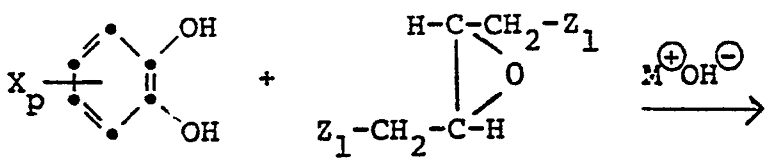
III,

worin $Z_1$ eine reaktionsfähige veresterte Hydroxygruppe bedeutet, und die anderen Symbole die oben angegebenen Bedeutungen haben. Man arbeitet vorzugsweise in Gegenwart von Verdünnungsmetteln, z.B. Niederalkylformamiden oder -sulfoxiden und bei Temperaturen zwischen ungefähr 50 und ungefähr 70°.

Eine reaktionsfähige veresterte Hydroxygruppe $Z_1$ ist vorzugsweise ein Halogenatom, insbesondere Chlor aber auch Brom oder Jod; oder eine aliphatische oder aromatische Sulfonyloxygruppe, z.B. Methansulfonyloxy-, Benzolsulfonlyoxy, Toluolsulfonyloxy- oder Brombenzolsulfonyloxygruppe.

Die so erhaltenen d,l - erythro - Verbindungen der Formel III, welche die R,S- und S,R-Enantiomere enthalten, können mit optisch aktiven Säuren, z.B. Mandelsäure, Weinsäure, Kampfersulfonsäure oder 1 - (1 - Naphthyl) - äthyl - isocyanat, getrennt werden. Eine solche Trennung kann vorzugsweise, wie in den Beispielen illustriert, gemäss der weiter unten beschriebenen Verfahrensvariante 3), oder gemäss J. Org. Chem. *43*, 3803(1978), durchgeführt werden. Ein Ausgangsstoff der Formel III kann auch durch chirale Synthese, wie hier unten illustriert, hergestellt werden. Man verwendet in dieser Synthese die billige, natürliche d - Weinsäure für den Aufbau des vier Kohlenstoffatome enthaltenden aliphatischen Teils des Ausgangsstoffes.

Ein weiteres Verfahren zur Herstellung von Verbindungen der Erfindung besteht darin, dass man

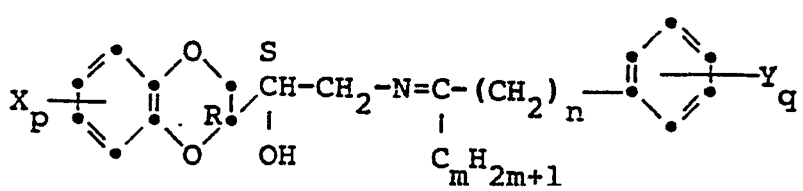
(VIII)

worin alle Symbole die oben angegebenenen Bedeutungen haben, mit komplexen chiralen Alkalimetallorganoboranen oder -alanen, chiral reduziert.

Die Reduktion wird in üblicher Weise, z.B. mit Lithium - $\beta$ - isopinocampheyl - 9 - borabicyclo[3,3,1]nonylhydrid oder mit Substitutionsprodukten von Lithiumaluminiumhydrid mit chiralen Basen, z.B. Ephedrin oder Amphetaminen, durchgeführt.

Die Schiff'schen Basen der Formel VIII können ausgehend von Aminen der allgemeinen Formel IX

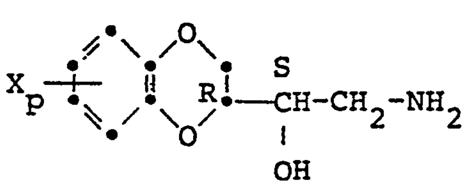
(IX)

und entsprechenden Ketonen hergestellt werden. Die genannten Amine können durch Umsetzung eines Epoxids der allgemeinen Formel III mit Ammoniak erhalten werden. Die Ketone sind bekannt, oder sie können aus bekannten Alkoholen der allgemeinen Formel X

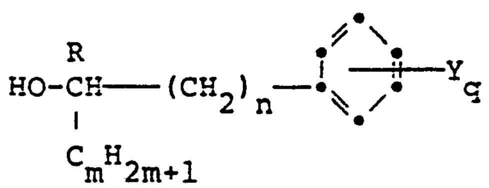 (X)

nach bekannten Methoden, z.B. durch Oxydation mit Chromsäure oder ihrem Anhydrid, hergestellt werden.

Schliesslich können die Verbindungen der Erfindung dadurch hergestellt werden, dass man

(3) eine d,l-erythro-2-Oxiranyl-1,4-benzodioxan-Verbindung der allgemeinen Formel XI

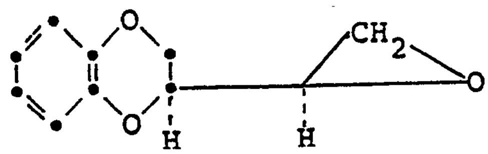 (XI)

mit einer l-R-Amino-Verbindung der allgemeinen Formel XII

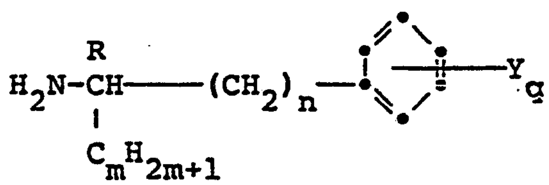 (XII),

in welchen alle Symbole die oben angegebenen Bedeutungen haben, umsetzt, die erhaltenen diastereomeren Verbindungen in ihre Säureadditionssalze unwandelt und das erythro - Gemisch von Salzen der diastereomeren, der Formel I entsprechenden Verbindungen, welches Gemisch die R,S,R- und S,R,R- Diastereomere enthält, durch selektive Kristallisation auftrennt.

Die Säureadditionssalze sind z.B. solche von pharmazeutisch verwendbaren, weiter unten genannten Säuren. So fällt z.B. das Hydrochlorid des gewünschten R,S,R,-Stereoisomeren bevorzugt aus der Lösung aus, während das unerwünschte S,R,R,-Stereoisomere in der Lösung bleibt. Wenn nötig, wird die Umkristallisation, bis die optische Drehung der erwünschten linksdrehenden Verbindung konstant bleibt, wiederholt.

Die Umkristallisation wird vorzugsweise in wasserfreien oder wässerigen Niederalkanolen, z.B. Aethanol, iso- oder n-Propanol, durchgeführt.

Die im ersten Schritt des Verfahrens 3) erhaltenen diastereomeren Produkte, welche durch Umsetzung des Ausgangsstoffes XI mit einer Verbindung XII entstehen, werden ähnlich wie im Verfahren 1) beschrieben, hergestellt. Man verwendet jedoch anstelle der optisch reinen R,S,-Verbindung der Formel III, ihre entsprechenden (erythro)-R,S und S,R-Gemische, welche gemäss den Verfahrensschritten a) bis c) wie oben beschrieben, erhalten werden können.

In einer Abänderung des Verfahrens 3) kann der erste Schritt, welcher als Zwischenprodukte die diastereomeren Verbindungen ergibt, in einer zum Verfahren 2) analogen Weise durchgeführt werden. Man setzt jedoch anstelle der optisch reinen R,S-Verbindung der Formel VIII, ihre entsprechenden (erythro) - R,S - und S,R-Gemische ein. Diese können gemäss den Verfahrensschritten a) bis c), dann durch Umsetzung mit Ammoniak und nachfolgend mit einem entsprechenden Keton, wobei man die als Ausgangsstoff der Formel VIII eingesetzte Schiff'sche Base erhält, hergestellt werden.

Die Verbindungen der Erfindung können in Form von freien Basen oder also Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxyd oder Ammoniumhydroxyd, basischen Salz oder einem Kationenaustauscher, z.B. mit einem Alkalimetallhydroxyd- oder -carbonat, umgewandelt werden. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Phenylessig-, Benzoe-, 4 - Aminobenzoe-, Anthranil-, 4 - Hydroxybenzoe-, Salicyl-, 4 - Aminosalicyl, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure. Diese oder andere Salze, z.B. die Pikrate, können auch in der Reinigung von freien Basen

verwendet werden. Die Basen werden in ihre Salze übergeführt, die Salze abgetrennt und die Basen aus den Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin Ausgangsstoff in Form eines Salzes oder optisch reinen Antipoden, verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien werden in erster Linie aus Fettemulsionen oder -suspensionen hergestellt. Die genannten Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die wenn erwünscht, weitere pharmakologisch wertvolle Stoffe enthalten können, werden in an sich bekannter Weise, z.B. mittels konventioneller Misch,- Granulier- oder Dragierverfahren, hergestellt und enthalten von etwa 0.1% bis etwa 90%, insbesondere von etwa 1% bis etwa 75% des Aktivstoffes.

Die folgenden Beispiele dienen zur Illustration der Erfindung. Temperaturen werden in Celsiusgraden angegeben, udn die Angaben über Teile betreffen Gewichtsteile. Wenn nicht anders definiert, wird das Eindampfen von Lösungsmitteln unter vermindertem Druck, vorzugsweise zwischen ungefähr 15 und 100 mmHg, durchgeführt.

Die oben genannten erythro-Epoxide der Formel III können wie folgt aufgezeichnet werden:

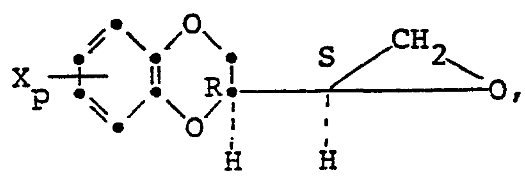

während die unerwünschten threo-Epoxide die folgende Formel aufweisen

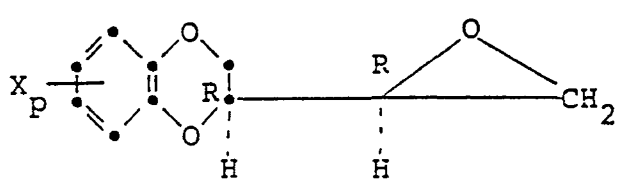

Beispiel 1

Ein Gemisch von 11 g d,l - erythro - 2 - Oxiranyl - 1,4 - benzodioxan und 9,2 g l - 3R - Amino - 1 - phenylbutan wird in einer Stickstoffatomsphäre 4 Stunden unter Rühren auf 100° erhitzt. Das noch warme Reaktionsgemisch wird in 100 ml 2-Propanol gelöst, die Lösung mit 17 ml 4-

normalen äthanolischem Chlorwasserstoff angesäuert und mit einigen Kristallen des erhaltenen Salzes geimpft. Das Gemisch wird bei Zimmertemperatur 5 Stunden und über Nacht bei —10° stehen gelassen. Der erhaltene Niederschlag wird abfiltriert und mit 25 ml kaltem 2-Propanol gewaschen. Man kocht unter Rückfluss 8,1 g des erhaltenen Niederschlags in 100 ml 2-Propanol und ungefähr 10 ml Wasser bis zur Auflösung. Die Lösung wird weider langsam auf —10° gekühlt, der erhaltene Niederschlag abfiltriert und mit Wasser und Diäthyläther gewaschen. Man erhält das l - erythro - 2R - [2 - (4 - Phenyl - 2R - butylamino) - 1S - hydroxyäthyl] - 1,4 - benzodioxanhydrochlorid, welches bei 200—202° schmilzt. $[\alpha]_D = -13,5°$ (in Dimethylsulfoxid).

Der Ausgangsstoff wird wie folgt hergestellt: Man lässt 2000 ml Wasser unter Rückfluss kochen und versetzt es unter heftigem Rühren zuerst mit 3 ml 40%-igem wässerigem Tetrabutylammoniumhydroxyd, 250 ml Salicylaldehyd und 280 ml trans-1,4-Dichlor-2-buten und dann tropfenweise mit einer Lösung von 83 g Natriumhydroxyd in 400 ml Wasser bis der pH-Wert des Gemisches 25 Minuten zwischen 7 und 8 bleibt. Dann wird das Gemisch ungefähr 6 Minuten, bis der pH-Wert 7 erreicht wird, unter Rückfluss gekocht, auf 20° gekühlt, und die wässerige Schicht mit Diäthyläther extrahiert. Der Extrakt wird mit der organischen Schicht vereinigt, mit 5%-iger wässeriger Natriumhydroxydlösung gewaschen, getrocknet und eingedampft. Der Rückstand wird destiliert und die bei 141—147°/0,7 mmHg siedende Fraktion aufgefangen. Man erhält den 2 - (4 - Chlor - trans - 2 - butenyloxy) - benzaldehyd.

Eine Lösung von 155,7 g der letztgenannten Verbindung in 3800 ml Methylenchlorid wird mit 357 g 85%-iger 3-Chlor-perbenzoesäure versetzt und das Gemisch eine Stunde gerührt und 4 Tage unter Rückfluss gekocht. Das Reaktionsgemisch wird gekühlt und filtriert. Das Filtrat wird mit Methylenchlorid und gesättigter wässeriger Natriumhydrogencarbonatlösung gewaschen, getrocknet und unter 40° eingedampft. Man erhält das 2 - (4 - Chlor - trans - 2,3 - epoxybutyloxy) - phenol - format, welches NMR-"Peaks" bei 3,2, 2,5, 4,1, 7,0 und 8,2 ppm aufweist.

Eine Lösung von 229,4 g der letztgenannten Verbindung in 900 ml Methanol wird unter Rühren in einer Stickstoffatmosphäre zwischen 8 und 15° tropfenweise mit 1100 ml einer 10%-igen wässerigen Kaliumhydroxydlösung versetzt. Das Rühren wird bei der genannten Temperatur und bei Zimmertemperatur über Nacht fortgesetzt, das Gemisch vom Rückstand dekantiert und mit Diäthyläther extrahiert. Der Extrakt wird mit dem Rückstand vereinigt, mit Wasser gewaschen, getrocknet und eingedampft. Der Rückstand wird aus Diäthyläther umkristallisiert. Man erhält das d,l - erythro - 2 - Oxiranyl - 1,4 - benzodioxan, welches bei 51—52° schmilzt.

Eine Lösung von 300 g l-Mandelsäure ($[\alpha]_D = -147,3°$ in Wasser) in 3500 ml wasserfreiem Aethanol wird unter Rühren zuerst mit 118,4 g Eisessig und dann mit 630,6 g 93,4%-igem d,l - 3 - Amino - 1 - phenylbutan versetzt, dessen Behälter mit 400 ml wasserfreiem Aethanol nachgewaschen wird. Das Gemisch wird 6 Stunden bei Zimmertemperatur und 20 Stunden bei 5° gerührt und dann filtriert. Der Rückstand wird mit 200 ml Petroläther gewaschen und in 1100 ml heissem Aethanol gelöst. Man läst die Lösung abkühlen und 19 Stunden bei Zimmertemperatur stehen, filtriert sie und wäscht den Rückstand mit 100 ml wasserfreiem Aethanol. Man erhält das d - 3 - Amino - 1 - phenyl - butan - l - mandelat, welches bei 140—142° schmilzt.

Man löst 228 g der letztgenannten Verbindung in einer minimalen Menge Wasser, stellt die Lösung basisch mit einer 15%-igen wässerigen Natriumhydroxydlösung und extrahiert sie mit Methylenchlorid. Der Extrakt wird mit Wasser gewaschen, getrocknet und eingedampft. Man erhält das l - 3R - Amino - 1 - phenyl - butan. $[\alpha]_D^{25} = -7,0°$ (in Aethanol). Sein Hydrochlorid zeigt einen $[\alpha]_D^{25}$-Wert von +7,23° (in Wasser), d.h. die Salzbildung kehrt die Drehungsrichtung um.

Die absolute Konfiguration des oben genannten 2R - [2 - (4 - Phenyl - 2R - butylamino) - 1S - hydroxyäthyl] - 1,4 - benzodioxanhydrochlorids kann wie folgt bestimmt werden: Seine Mutterlaugen, welche seine erwartete S,R,R-diastereomere Form enthalten [die Konfiguration seines 4 - Phenyl - 2R - butylamino - Teils ist bereits in Rec. Trav. Chim. *82*, 189 (1963) aufgeklärt worden], werden eingedampft. Man löst 1 g des Rückstandes in 20 ml Aethanol und 10 ml Wasser. Die Lösung wird mit einer Lösung von 0,85 g Natrium-meta-perjodat im 10 ml Wasser versetzt und das Gemisch 24 Stunden bei Zimmertemperatur stehen gelassen. Es wird filtriert, das Filtrat eingedampft und der Rückstand in 20 ml Aethanol aufgenommen. Die Lösung wird mit 0,2 g Natriumborhydrid versetzt und das Gemisch eine Stunde bei Zimmertemperatur gerührt. Es wird eingedampft, der Rückstand in 100 ml Diäthyläther und 20 ml Essigsäureäthylester aufgenommen und die Lösung mit normaler Chlorwasserstoffsäure gewaschen. Die organische Schicht wird getrocknet, eingedampft und der Rückstand bei 160° gas-chromatographiert. Man erhält das 2R - Hydroxymethyl - 1,4 - benzodioxan, welches einen $[\alpha]_D^{25}$-Wert von +33.4° (in Aethanol) aufweist. Seine absolute Konfiguration ist in J. Med. Chem. *20*, 880 (1977) beschrieben. Berücksichtigt man die Sequenz-Regeln [Experientia *12*, 81 (1956)], enthält die oben genannte Mutterlauge das d - erythro - 2S - [2 - (4 - Phenyl - 2R - butylamino) - 1R - hydroxyäthyl] - 1,4 - benzodioxan - hydrochlorid, dessen der Formel I entsprechende diastereomere Form das R,S,R,-Stereoisomere sein muss.

## Beispiel 2

Ein Gemisch von 3,75 g d,l - erythro - 2 - Oxiranyl - 1,4 - benzodioxan und 3,5 g 2R - Amino - 1 - p - methoxy - phenyl - propan [J. Med. Chem. *16*, 480 (1973)] wird unter Rühren 4

Stunden auf 100° erhitzt. Das Reaktionsgemisch wird in 25 ml Isopropanol gegossen, die Lösung mit 4-normalem Chlorwasserstoff in Aethanol angesäuert, der Niederschlag abgetrennt und aus Isopropanol umkristallisiert. Man erhält das I - erythro - 2R - [2 - (3 - p - Methoxy - phenyl - 2R - propylamino) - 1S - hydroxyäthyl] - 1,4 - benzodioxan - hydrochlorid, welches bei 161—163° schmilzt. $[\alpha]_D^{25} = -39,2°$ (in Dimethylsulfoxid).

Beispiel 3

Gemäss der in den vorhergehenden Beispielen beschriebenen Methode, ausgehend von äquivalenten Mengen entsprechender Ausgangsstoffe, werden die folgenden Verbindungen der Formel I hergestellt ($X = H$, $m = q = 1$):

| No. | n | Y | Salz |
|---|---|---|---|
| 1 | 1 | H | Hydrochlorid |
| 2 | 1 | o-$OCH_3$ | Methansulfonat |
| 3 | 2 | p-$OCH_3$ | Hydrochlorid |
| 4 | 2 | p-F | Hydrochlorid |

Beispiel 4

Ein Gemisch von 3 g d,l - erythro - 2 - Oxiranyl - 1,4 - benzodioxan und 2,8 g R - 3 - (m-Methoxyphenyl) - 2 - propylamin wird 3 Stunden bei 100° gerührt. Nach Abkühlen wird es in 10 ml Isopropanol gelöst und die Lösung mit 4-normalen Chlorwasserstoff in Aethanol angesäuert. Nach Zugabe von Diäthyläther kristillisiert das 1:1-Gemisch von R-S-R- und S-R-R - 2 - [2 - (3 - m - Methoxyphenyl - 2 - propylamino) - 1 - hydroxyäthyl] - 1,4 - benzodioxan - hydrochlorid aus, welches bei 110—115° schmilzt und wie vorher gezeigt, auftrennbar ist.

Der Ausgangsstoff wird wie folgt hergestellt: Ein Gemisch von 33,5 g m-Methoxy-phenylaceton, 25 g d - $\alpha$ - Methylbenzylamin und 120 ml Benzol wird 24 Stunden unter Rückfluss gekocht, wobei man das entstandene Wasser (3,5 ml) azeotropisch abtrennt. Das Reaktionsgemisch wird eingedampft, der Rückstand in 20 ml Aethanol gelöst und die Lösung über 20 g Raney-Nickel bei 50° und 3,1 Atmosphären bis zur Aufnahme von einem Moläquivalent Wasserstoff hydriert. Das Gemisch wird filtriert, das Filtrat eingedampft, der Rückstand in 100 ml Isopropanol aufgenommen und die Lösung mit äthanolischem Chlorwasserstoff angesäuert. Der erhaltene Niederschlag wird dreimal, bis zur Erreichung einer konstanten Drehung und eines konstanten Schmelzpunktes, aus Aethanol-Isopropanol umkristalisiert. Man erhält das N - d - $\alpha$ - Methylbenzyl - m - methoxyphenyl - 2 - propylamin - hydrochlorid, $[\alpha]_D^{25} = +19,16°$ (2% in Methanol), welches bei 230° unter Zersetzung schmilzt.

Eine Lösung von 16 g der letztgenannten Verbindung in 120 ml Aethanol und 30 ml Wasser wird über 5 g eines 10%-igen Palladium-auf-Kohle-Katalysators bis zur Aufnahme von einem Moläquivalent Wasserstoff hydriert. Das Gemisch wird filtriert, eingedampft, der Rückstand in Wasser aufgenommen und die Lösung mit Natriumhydroxyd basisch gemacht. Es wird mit Methylenchlorid extrahiert, der Extrakt getrocknet und eingedampft. Man erhält das R - 3 - (m - Methoxyphenyl) - 2 - propylamin als ein Oel.

Beispiel 5

Ein Gemisch von 2,45 g d,l - erythro - 2 - Oxiranyl - 1,4 - benzodioxan und 2,3 g R - 3 - (p - Fluorphenyl) - 2 - propylamin wird 3 Stunden bei 100° gerührt. Nach Abkühlen wird das Reaktionsgemisch in 20 ml heissem Isopropanol gelöst und die Lösung mit 4-normalen Chlorwasserstoff in Aethanol angesäuert. Das Gemisch wird langsam gekühlt, filtriert und der Rückstand aus wässerigem Isopropanol umkristallisiert. Man erhält das I - erythro - 2R - [2 - (3 - p - Fluorphenyl - 2R - propylamino) - 1S - hydroxyäthyl] - 1,4 - benzodioxan - hydrochlorid, welches bei 238—240° unter Zersetzung schmilzt. $[\alpha]_D^{25} = -11,97°$ (2% in Dimethylsulfoxid).

Der Ausgangsstoff wird wie folgt hergestellt: Eine Lösung von 10 g des racemischen R - S - 3 - (p - Fluorphenyl) - 2 - propylamins in 50 ml heissem Isopropanol wird mit 12 g I - 2 - Benzodioxanyl - essigsäure versetzt. Der nach Abkühlen erhaltene Niederschlag wird abfiltriert und aus Isopropanol umkristallisiert. Das Produkt wird in einer minimalen Menge Wasser aufgelöst, die Lösung mit Natriumhydroxyd basisch gemacht und mit Methylenchlorid extrahiert. Der Extrakt wird getrocknet und eingedampft. Man erhält das R - 3 - (p - Flurophenyl) - 2 - propylamin. Sein Hydrochlorid schmilzt bei 142°. $[\alpha]_D^{25} = +6,25°$ (2% in Wasser).

Beispiel 6

Herstellung von 10'000 Tabletten mit einem Gehalt von je 10 mg der aktiven Substanz:

*Bestandtiele:*

| | |
|---|---|
| 2R-[2-(4-Phenyl-2R-butylamino)-1S-hydroxyäthyl]-1,4-benzodioxanhydrochlorid | 100 g |
| Milchzucker | 1706 g |
| Maisstärke | 90 g |
| Polyäthylenglykol 6000 | 90 g |
| Talkpulver | 90 g |
| Magnesiumstearat | 24 g |
| Gereinigtes Wasser | q.s. |

*Verfahren:* Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 45 ml Wasser suspendiert und die Suspension zu der siedenden Lösung von Polyäthylenglykol in 180 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 7,1 mm Durchmesser, welche eine Bruchrille aufweisen, gepresst.

Beispiel 7

Herstellung von 10'000 Kapseln mit einem Gehalt von 25 mg der aktiven Substanz:

*Bestandteile:*

| | |
|---|---|
| 2R-[2-(3-p-Methoxyphenyl-2R-propylamino)-1S-hydroxyäthyl]-1,4-benzodioxan-hydrochlorid | 250 g |
| Milchzucker | 2350 g |
| Talkpulver | 150 g |

*Verfahren:* Sämtliche pulverigen Bestandteile werden mit einem Sieb von 0,6 Maschenweite gesiebt. Dann wird der Wirkstoff zuerst mit Talk und darauffolgend mit dem Milchzucker in einem geeigneten Mischer homogenisiert. Kapseln Nr. 2 werden mit je 300 mg des Gemisches mit einer Füllmaschine gefüllt.

Beispiel 8

Ein Gemisch von 1,1 g erythro - 2R - (1S - Oxiranyl) - 1,4 - benzodioxan und 0,92 g 3R - Amino - 1 - phenylbutan wird unter Rühren in einer Stickstoffatmosphäre 4 Stunden auf 100° erhitzt. Das Reaktionsgemisch wird dann in einer minimalen Menge 2-Propanol gelöst, die Lösung mit 4-normaler Chlorwasserstoffsäure in wasserfreiem Aethanol angesäuert und bei —10° über Nacht stehen gelassen. Der erhaltene Niederschlag wird abfiltriert, mit kaltem 2-Propanol und Diäthyläther gewaschen. Man erhält das erythro - 2R - [2 - (4 - Phenyl - 2R - butylamino) - 1S - hydroxyäthyl] - 1,4 - benzodioxan - hydrochlorid, welches bei 200—202° schmilzt. $[\alpha]_D^{25} = -13,5°$. Das Produkt ist mit demjenigen des Beispiels 1 identisch.

Der Ausgangsstoff wird wie folgt hergestellt: Eine Lösung von 8 g Thiophenol in 150 ml Tetrahydrofuran wird unter Kühlen im Eisbad mit 4 g 50%-igem Natriumhydrid in Mineralöl unter Rühren in einer Stickstoffatmosphäre versetzt. Nach dem Aufhören der Wasserstoffentwicklung gibt man 12,8 g d,l - erythro - 2 - Oxiranyl - benzodioxan dazu und lässt das Gemisch sich auf Zimmertemperatur erwärmen. Das Reaktionsgemisch wird dann 2 Stunden unter Rückfluss gekocht, abgekühlt, mit 10%-iger Chlorwasserstoffsäure angesäuert und eingedampft. Der Rückstand wird in Diäthyläther aufgenommen, die Lösung mit Wasser gewaschen und eingedampft. Man erhält das d,l - erythro - 2 - (2 - Phenylthio - 1 - hydroxyäthyl) - 1,4 - benzodioxan. Dieses wird in 50 ml Toluol, welches 1% N,N-Dimethyläthanolamin enthält, aufgenommen, die Lösung mit 14 g (R) - N - [1 - (1 - Naphthyl) - äthyl] - isocyanat versetzt und 6 Stunden unter Rückfluss gekocht. Das Reaktionsgemisch wird eingedampft und der Rückstand auf neutralem Aluminiumoxid chromatographiert, wobei man mit Hexan-Methylenchlorid (5:1) auf einer Säule von 280 mm eluiert. Die ersten Fraktionen werden gesammelt und eingedampft. Man erhält das N - [1R - (1 - Naphthyl) - äthyl] - 1S - (1,4 - benzodioxan - 2R - yl) - 2 - phenylthioäthylcarbamat.

Eine Lösung von 18,8 g der letztgenannten Verbindung in 200 ml Methylenchlorid und 10,2 g Triäthylamin wird tropfenweise mit einer Lösung von 3,4 ml Trichlorsilan in 50 ml Methylenchlorid versetzt und das Reaktionsgemisch über Nacht unter Rückfluss gekocht. Nach Abkühlung wird es mit gesättigter wässeriger Ammoniumchloridlösung gewaschen und eingedampft. Man erhält das erythro - 2R - (2 - Phenylthio - 1S - hydroxyäthyl) - 1,4 - benzodioxan.

Man löst die letztgenannte Verbindung in 60 m Methylenchlorid und versetzt die Lösung unter Rühren mit 4 g Trimethyloxonium-fluoroborat. Das Rühren wird bis zur Auflösung der ganzen Salzmenge fortgesetzt (3 Stunden). Das Gemisch wird dann unter Rühren mit 47 ml einer 10%-igen wässerigen Natriumhydroxydlösung versetzt, nach 3 Stunden die organische Schicht abgetrennt, getrocknet und eingedampft. Man erhält 3,4 g des gewünschten erythro - 2R - (1S - Oxiranyl) - 1,4 - benzodioxans, welches eine genügende Reinheit aufweist.

Der Ausgangsstoff kann auch gemäss der folgenden chiralen Synthese hergestellt werden. Dabei verwendet man die vier Kohlenstoffatome der billigen, natürlichen d-(R,R)-Weinsäure für die Ausbildung des vier Kohlenstoffatome enthaltenden aliphatischen Teils des Ausgangsstoffes:

Eine Lösung von 6,5 g o-Benzyloxyphenol und 1,8 g Natrium-methoxid in 100 ml Isopropanol wird mit 15 g 2,2 - Dimethyl - 1,3 - (4S,5S) - dioxolan - 4,5 - dimethanol - bis-tosylat [hergestellt gemäss D. Seebach, Helv. *60*, 301 (1977)] versetzt. Das Reaktionsgemisch wird 48 Stunden unter Rückfluss gekocht und eingedampft. Der Rückstand wird in Methylenchlorid aufgenommen, die Lösung mit Wasser gewaschen und eingedampft. Man erhält das 5 - (o - Benzyloxyphenoxymethyl) - 2,2 - dimethvl - 1,3 - (4S,5S) - dioxolan - 4 - methanol - tosylat.

Ein Gemish von 25 g der letztgenannten Verbindung, 60 ml 2-normaler Chlorwasserstoffsäure und 120 ml Methanol wird 6 Stunden unter Rückfluss gekocht und teilweise eingedampft. Das Konzentrat wird mit Wasser verdünnt und mit Methylenchlorid extrahiert. Der Extrakt wird mit wässeriger Natriumhydrogencarbonatlösung gewaschen, getrocknet und eingedampft. Der Rückstand wird aus Isopropanol umkristallisiert. Man erhält das reine 1 - O - (o - Benzyloxyphenyl) - L - threitol, welches bei 105° schmilzt.

Eine Lösung von 10 g der letztgenannten Verbindung in 100 ml Methylenchlorid wird zuerst mit 0,2 g Methansulfonsäure und dann langsam mit 5,1 g Isopropenyläther in 20 ml Methylenchlorid versetzt, wobei man das Gemisch bei 0,5° 3 Stunden rührt. Das Reaktionsgemisch wird weitere 4 Stunden bei Zimmertemperatur gerührt, dann mit Triäthylamin neutralisiert, mit Wasser gewaschen und eingedampft. Man erhält das 1 - O - (o - Benzyloxyphenyl) - 3,4 - isopropyliden - L - threitol.

Die letztgenannte Verbindung wird in 50 ml Pyridin bei 5° aufgenommen, langsam mit 4 g Methansulfonylchlorid versetzt und das Gemisch über Nacht bei Zimmertemperatur stehen gelasen. Das Reaktionsgemisch wird in Eiswasser gegossen, die Suspension filtriert und der Rückstand mit Wasser gewaschen. Man erhält das 1 - O - (o - Benzyloxyphenyl) - 3,4 - isopropyliden - L - threitol - 2 - mesylat.

Die letztgenannte Verbindung wird in 100 ml Aethanol aufgenommen und über 1 g 10%-igem Palladium-auf-Kohle-Katalysator bei 2,7 Atmosphären bis zur Aufnahme von einem Moläquivalent Wasserstoff hydriert. Das Gemisch wird filtriert, das Filtrat mit 2,5 Natrium-methoxid versetzt und das Gemisch 48 Stunden unter Rückfluss gekocht. Das Reaktionsgemisch wird mit konz. Chlorwasserstoffsäure neutralisiert, eingedampft und der Rückstand in einem Gemisch von 30 ml Chlorwasserstoffsäure und 60 ml Methanol 5 Stunden unter Rückfluss gekocht. Das Reaktionsgemisch wird auf ein kleines Volumen konzentriert und mit Methylenchlorid extrahiert. Der Extrakt wird mit wässeriger Natriumhydrogencarbonatlösung gewaschen, getrocknet und eingedampft. Man erhält das 1S - (1,4 - Benzodioxan - 2R - yl) - äthylenglykol.

Die letztgenannte Verbindung wird in 30 ml Orthoessigsäure-triäthylester gelöst, mit 0,2 ml Trifluoressigsäure versetzt und das Gemisch 6 Stunden bei Zimmertemperatur stehen gelassen. Es wird mit Triäthylamin neutralisiert, eingedampft, der Rückstand in Wasser aufgenommen und das Gemisch mit Methylenchlorid extrahiert. Der Extrakt wird getrocknet und eingedampft. Man erhält das entsprechende Orthoacetat.

Die letztgenannte Verbindung wird in 50 ml Methylenchlorid aufgelöst und mit 10 ml Trimethylchlorsilan versetzt. Nach Rühren von 4 Stunden wird das Gemisch bei Zimmertemperatur eingedampft, der Rückstand in 120 ml Methylenchlorid aufgenommen, mit 4 g Tetrabutylammoniumbromid und 120 ml 2-normaler wässeriger Natriumhydroxydlösung versetzt. Das Gemisch wird eine Stunde stark gerührt, die organische Schicht abgetrennt, mit Wasser gewaschen, getrocknet und eingedampft. Man erhält das gewünschte erythro - 2R - (1S - Oxiranyl) - 1,4 - benzodioxan für die oben genannte Umsetzung mit 3R-Amino-1-phenylbutan.

Das rohe R,S-Epoxid wird in einem kurzen Kugelrohr-Destillationsapparat bei 130°/0,1 mmHg destilliert. Man erhält das reine, R,S-Epoxid, welches bei 65° schmilzt. Die optische Drehung $[\alpha]_D^{25} = -48,6°$ (c = 2% in Methylenchlorid).

Beispiel 9

Ein Gemisch von 10 g erythro - 2R - (1S - Oxiranyl) - 1,4 - benzodioxan und 100 ml 10%-igem Ammoniak in Aethanol wird in einem Einschmelzrohr 12 Stunden auf 100° erhitzt. Nach Abkühlen wird das Reaktionsgemisch eingedampft, der Rückstand in 100 ml Tetrahydrofuran aufgelöst und es werden 8,3 g Benzylaceton zusammen mit 40 g 3A-Molekularsieb dazugegeben. Das Gemisch wird über Nacht gerührt, filtriert, mit 100 ml Tetrahydrofuran verdünnt und dann in einer Stickstoffatmosphäre bei 25° mit 19 g Lithium - β - isopinocampheyl - 9 - borabicyclo[3,3,1]nonyl - hydrid 24 Stunden gerührt. (Der Katalysator ist gemäss S. Krishnamurthy et al., Abstracts of Papers, 173rd ACS Meeting, March 20—25, 1977; No. ORGN. 17, hergestellt worden.) Das Reaktionsgemisch wird nachher mit 25 ml 4-normaler wässeriger Chlorwasserstoffsäure behandelt und eingedampft. Der Rückstand wird mit 100 ml warmem Isopropanol gerührt, filtriert und der Rückstand aus wässerigem Isopropanol umkristallisiert. Man erhält das erythro - 2R - [2 - (4 - Phenyl - 2R - butylamino) - 1S - hydroxyäthyl] - 1,4 - benzodioxan - hydrochlorid, welches bei 200—202° schmilzt. Das Produkt ist mit demjenigen des Beispiels 1 identisch.

**Patentansprüche**

1. l - erythro - 2 - (2 - Aralkylamino - 1 - hydroxyäthyl) - 1,4 - benzodioxane der allgemeinen Formel I

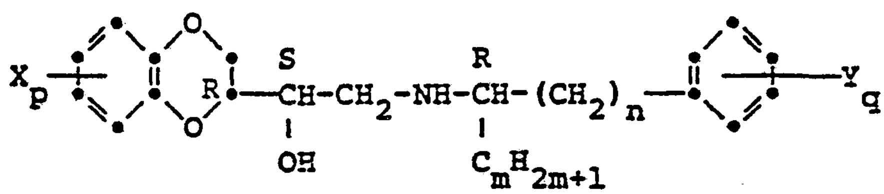

(I),

worin jedes der Symbole X und Y Wasserstoff, Niederalkyl, Niederalkoxy, Halogen oder Trifluormethyl bedeutet, jedes der Symbole p und q für die ganze Zahl 1 oder 2 steht, und jedes der Symbole m und n die ganze Zahl 1 bis 4 bedeutet und ihre Salze.

2. Verbindungen der im Anspruch 1 angegebenen Formel I, worin jedes der Symbole X und Y Wasserstoff, Fluor, Chlor, Trifluormethyl, oder Alkyl oder Alkoxy, jedes mit höchstens 4 Kohlenstoffatomen, bedeutet, p für die Zahl eins steht, q 1 oder 2 bedeutet und jedes der Symbole m und n für die Zahl 1 oder 2 steht, und ihre phramazeutisch verwendbaren Säureadditionssalze.

3. Verbindungen gemäss Anspruch 1 der allgemeinen Formel II

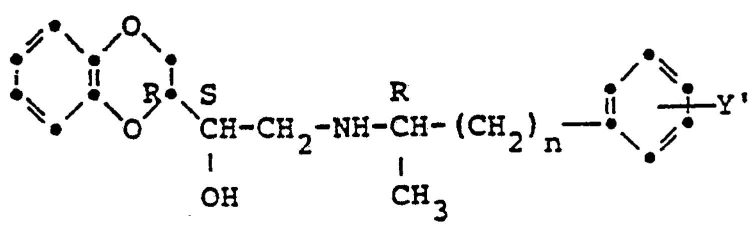

(II),

worin Y′ Wasserstoff, Alkyl oder Alkoxy, jedes mit höchstens 4 Kohlenstoffatomen, bedeutet, und n für die Zahl 1 oder 2 steht, und ihre pharmazeutisch verwendbaren Säureadditionssalze.

4. l - erythro - 2R - [2 - (4 - Phenyl - 2R - butylamino) - 1S - hydroxyäthyl] - 1,4 - benzodioxan und seine pharmazeutisch verwendbaren Säureadditionssalze.

5. l - erythro - 2R - [2 - (3 - p - Methoxyphenyl - 2R - propylamino) - 1S - hydroxyäthyl] - 1,4 - benzodioxan und seine pharmazeutisch verwendbaren Säureadditionssalze.

6. Pharmazeutische Präparate enthaltend eine Verbindung gemäss einem der Ansprüche 1 bis 5.

7. Die Verbindungen der Ansprüche 1 bis 5 zur Verwendung also Pharmazeutika.

8. Verfahren zur Herstellung von einem spezifischen Diastereomeren von erythro - 2 - (2 - Aralkylamino - 1 - hydroxyäthyl) - 1,4 - benzodioxanen der allgemeinen Formel I

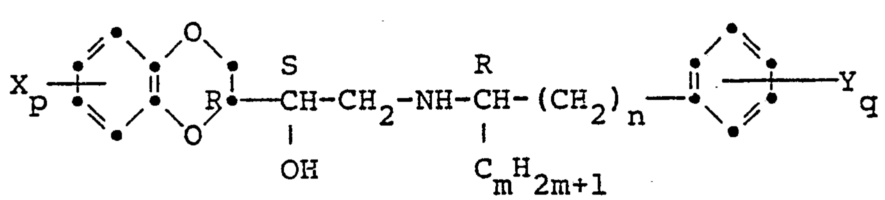

(I),

worin jedes der Symbole X und Y Wasserstoff, Niederalkyl, Niederalkoxy, Halogen oder Trifluormethyl bedeutet, jedes der Symbole p und q für die ganze Zahl 1 oder 2 steht, und jedes der Symbole m und n die ganze Zahl 1 bis 4 bedeutet und ihren Salzen, dadurch gekennzeichnet, dass man

1) Verbindungen der allgemeinen Formeln III und IV

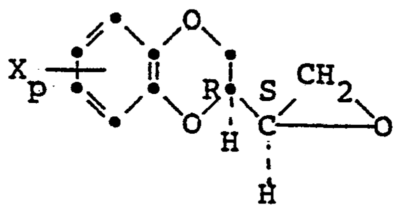 + 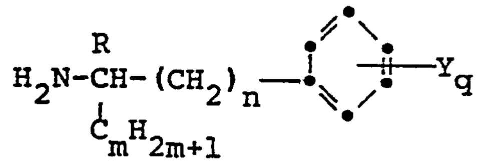

(III) (IV),

in welchen alle Symbole die oben angegebenen Bedeutungen haben, kondensiert oder

2) Verbindungen der allgemeinen Formel VIII

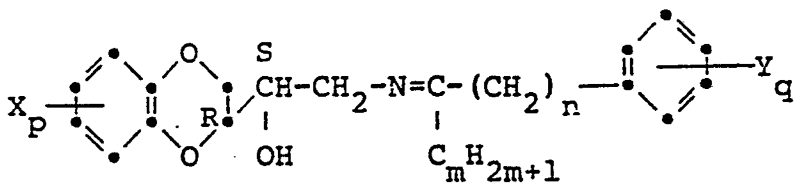

(VIII)

worin alle Symbole die oben angegebenen Bedeutungen haben, mit komplexen chiralen Alkalimetall-organoboranen oder -alanen, chiral reduziert oder

3) eine d,l-erythro-2-Oxiranyl-1,4-benzodioxan-Verbindung der allgemeinen Formel XI

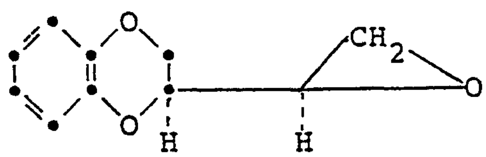

(XI)

mit einer l-R-Amino-Verbindung der allgemeinen Formel XII

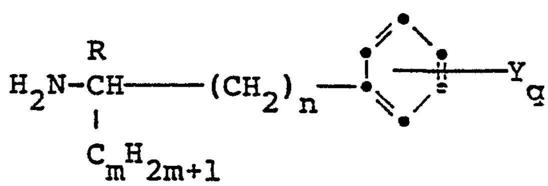

(XII),

in welchen alle Symbole die oben angegebenen Bedeutungen haben, umsetzt, die erhaltenen diastereomeren Verbindungen in ihre Säureadditionssalze umwandelt und das erythro-Gemisch von Salzen der diastereomeren, der Formel I entsprechenden Verbindungen, welches Gemisch die R,S,R,- und S,R,R-Diastereomere enthält, durch selektive Kristallisation auftrennt oder

4) ein (erythro)-R,S- und -S,R-Gemisch einer der oben gezeigten Formel VIII entsprechenden Verbindung, worin alle Symbole die oben angegebenen Bedeutungen haben, mit komplexen chiralen Alkalimetall-organoboranen oder -alanen, chiral reduziert, die erhaltenen diastereomeren Verbindungen in ihre Säureadditionssalze umwandelt und das erythro-Gemisch von Salzen der diastereomeren, der Formel I entsphrechenden Verbindungen, welches Gemisch die R,S,R- und S,R,R-Diastereomere enthält, durch selektive Kristallisation auftrennt und, wenn erwünscht, eine erhaltene freie Verbindung in ein Salz oder ein erhaltenes Salz in die freie Verbindung oder in ein anderes Salz überführt.

**Revendications**

1. l - érythro - 2 - (2 - aralkylamino - 1 - hydroxyéthyl) - 1,4 - benzodioxannes de formule générale I

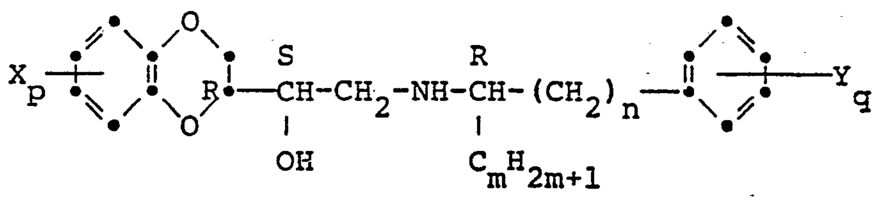

(I),

dand laquelle chacun des symboles X et Y représente l'hydrogène, un groupe alkyle inférieur, alcoxy inférieur, un halogène ou le groupe trifluorométhyle; chacun des symboles p et q représente le nombre entier 1 ou 2 et chacun des symboles m et n un nombre entier allant de 1 à 4, et leurs sels.

2. Composés de formule I selon la revendication 1, dans laquelle chacun des symboles X et Y représente l'hydrogène, le fluor, le chlore, un groupe trifluorométhyle, ou un groupe alkyle ou alcoxy, avec chacun 4 atomes de carbone au maximum, p est égal à 1, q est égal à 1 ou 2 et chacun des symboles m et n est égal à 1 ou 2, et leurs sels formés par addition avec des acides acceptables pour l'usage pharmaceutique.

3. Composés selon la revendication 1, répondant à la formule générale II

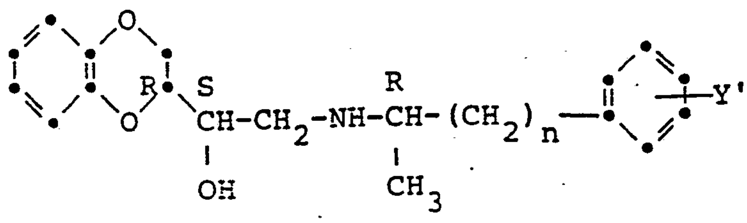

(II),

dans laquelle Y' représente l'hydrogène, un groupe alkyle ou alcoxy contenant chacun 4 atomes de carbone au maximum, n est égal à 1 ou 2, et leurs sels formés par addition avec des acides acceptables pour l'usage pharmaceutique.

4. l - érythro - 2R - /2 - [4 - phényl - 2R - butylamino] - 1S - hydroxyéthyl/ - 1,4 - benzodioxanne et ses sels formés par addition avec des acides acceptables pour l'usage pharmaceutique.

5. l - érythro - 2R - /2 - [3 - p - méthoxyphényl - 2R - propylamino] - 1S - hydroxyéthyl/ - 1,4 - benzodioxanne et ses sels formés par addition avec des acides acceptables pour l'usage pharmaceutique.

6. Préparations pharmaceutiques contenant un composé selon l'une des revendications 1 à 5.

7. Les composés des revendications 1 à 5 pour l'utilisation en tant que produits pharmaceutiques.

8. Procédé de préparation d'un diastéréoisomère spécifique d'érythro - 2 - (2 - aralkylamino - 1 - hydroxyéthyl) - 1,4 - benzodioxannes répondant à la formule générale I

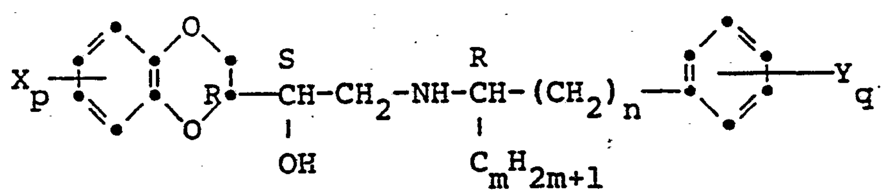

(I),

dans laquelle chacun des symboles X et Y représente l'hydrogène, un groupe alkyle inférieur, alcoxy inférieur, un halogène ou le groupe trifluorométhyle, chacun des symboles p et q représente le nombre entier 1 ou 2 et chacun des symboles m et n représente un nombre entier de 1 à 4, et de leurs sels, ce procédé se caractérisant en ce que:

1) on condense les composés répondant aux formules générales III et IV

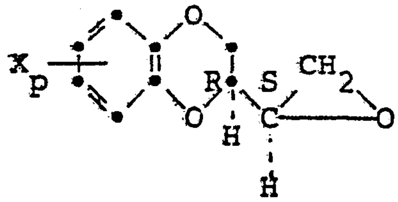 + 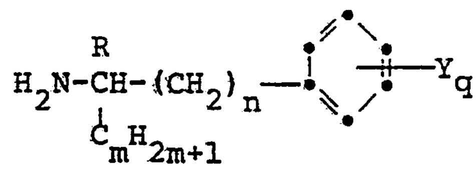

(III) (IV).

dans lesquelles tous les symboles ont les significations indiquées ci-dessus, ou bien

2) on soumet à réduction chirale des composés répondant à la formule générale VIII

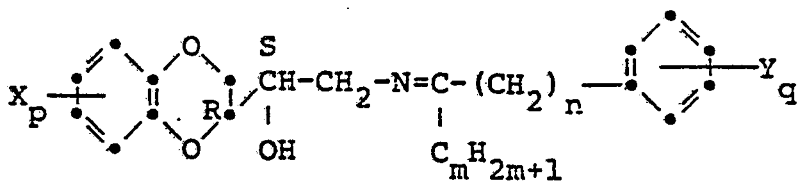

(VIII)

dans laquelle tous les symboles ont les significations indiquées ci-dessus, à l'aide d'organo-boranes ou -alanes de métaux alcalins complexes chiraux, ou bien

3) on fait réagir un composé de d,l-érythro-2-oxirannyl-1,4-benzodioxanne de formule générale XI

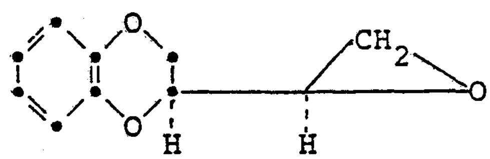

(XI)

avec un composé l-R-aminé de formule générale XII

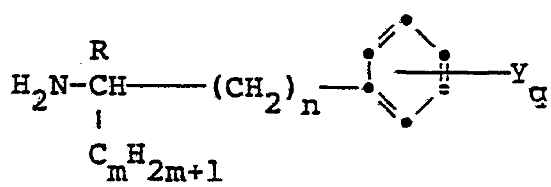

(XII),

tous les symboles ayant les significations indiquées ci-dessus, on convertit les composés diastéréoisomères obtenus en sels formés par addition avec des acides et on résout par cristallisation sélective le mélange érythro de sels des composés diastéréoisomères répondant à la Formule I, qui contient un mélange des diastéréoisomères R,S,R et S,R,R, ou bien

4) on soumet à réduction chirale à l'aide d'organo-boranes ou -alanes de métaux alcalins complexes chiraux un mélange (érythro)-R,S et -S,R d'un composé répondant à la formule VIII ci-dessus dans laquelle tous les symboles ont les significations indiquées, on convertit les composés diastéréoisomères obtenus en sels formés par addition avec des acides et on résout par cristallisation sélective le mélange érythro de sels des composés diastéréoisomères répondant à la formule I, qui contient un mélange des diastéréoisomères R,S,R et S,R,R, après quoi, si on le désire, on convertit un composé libre obtenu en un sel ou un sel obtenu en le composé libre ou en un autre sel.

**Claims**

1. A l - erythro - 2 - (2 - aralkylamino - 1 - hydroxyethyl) - 1,4 - benzodioxan of the general formula I

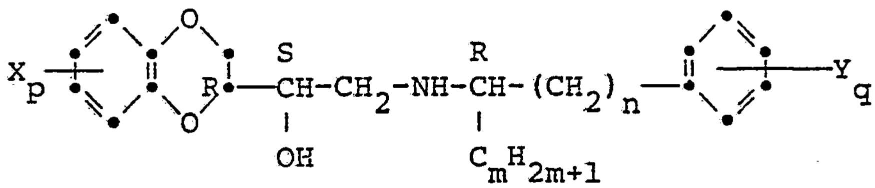

(I),

wherein each of X and Y is hydrogen, lower alkyl, lower alkoxy, halogen or trifluoromethyl, each of p and q is 1 or 2 and each of m and n is an integer from 1 to 4, or a salt thereof.

2. A compound of the formula I, as indicated in claim 1, wherein each of X and Y is hydrogen, fluorine, chlorine, trifluoromethyl, alkyl or alkoxy each containing at most 4 carbon atoms, p is 1, q is 1 or 2 and each of m and n is 1 or 2, or a pharmaceutically acceptable acid addition salt thereof.

3. A compound according to claim 1 of the general formula II

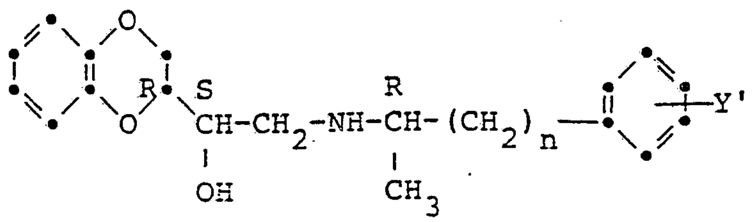

(II),

wherein Y' is hydrogen, alkyl or alkoxy, each containing at most 4 carbon atoms and n is 1 or 2, or a pharmaceutically acceptable acid addition salt thereof.

4. l - erythro - 2R - [2 - (4 - Phenyl - 2R - butylamino) - 1S - hydroxyethyl] - 1,4 - benzodioxan, or a pharmaceutically acceptable acid addition salt thereof.

5. l - erythro - 2R - [2 - (3 - p - Methoxyphenyl - 2R - propylamino) - 1S - hydroxyethyl] - 1,4 - benzodioxan, or a pharmaceutically acceptable acid addition salt thereof.

6. A pharmaceutical preparation comprising a compound as claimed in any one of claims 1 to 5.

7. A compound as claimed in any one of claims 1 to 5 for use as a pharmaceutical.

8. A process for the manufacture of a specific diastereoisomer of an erythro - 2 - (2 - aralkylamino - 1 - hydroxyethyl) - 1,4 - benzodioxan of the general formula I

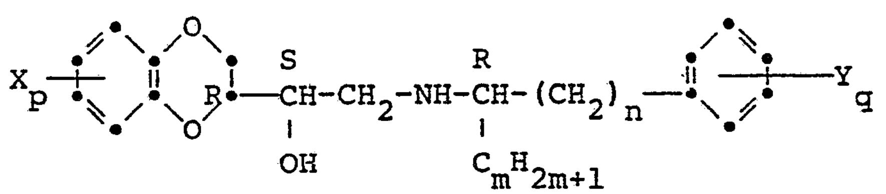

(I),

wherein each of X and Y is hydrogen, lower alkyl, lower alkoxy, halogen or trifluoromethyl, each of p and q is 1 or 2 and each of m and n is an integer from 1 to 4, or a salt thereof, which process comprises

1) condensing compounds of the general formulae III and IV

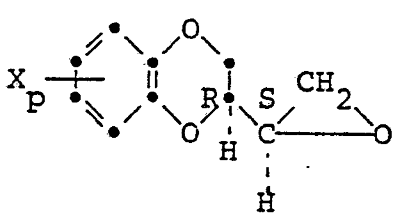

+

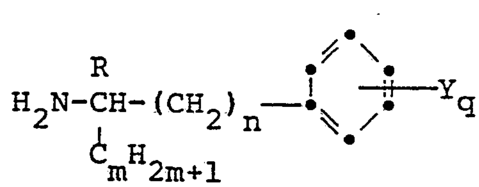

(III) (IV),

wherein all the symbols have the meanings given above or

2) chiral reduction of compounds of the formula VIII

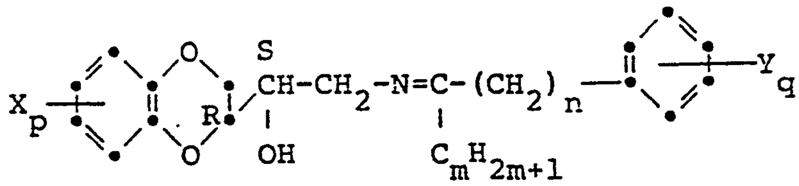

(VIII)

in which all the symbols have the meanings given above, with complex, chiral alkali metal organoboranes or -alanes or

3) reacting a d,l-erythro-2-oxiranyl-1,4-benzodioxan of the general formula XI

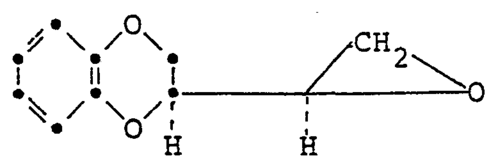

(XI)

with an l-R-amino-compound of the general formula XII

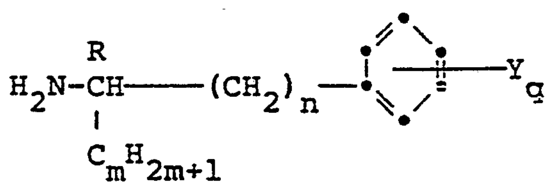

(XII),

wherein all the symbols have the meanings given above, converting the resultant diastereoisomers into their acid addition salts and resolving the erythro-mixture of salts of the diastereoisomers of the formula I, which mixture comprises the R,S,R- and S,R,R-diastereoisomers, by selective crystallization, or
4) chiral reduction of the (erythro)-R,S- and -S,R-mixture of a compound corresponding to the formula VIII above, wherein all the symbols have the meanings given above, with complex, chiral alkali metal organoboranes or -alanes, converting the resultant diastereoisomers into their acid addition salts and resolving the erythro-mixture of salts of the diastereoisomers of the formula I, which mixture comprises the R,S,R- and S,R,R-diastereoisomers, by selective crystallization, and, if desired, converting a resultant free compound into a salt or a resultant salt into the free compound or into another salt.